# EUROPEAN PATENT SPECIFICATION

(11) **EP 2 453 952 B1**
(45) Date of publication and mention of the grant of the patent: **14.02.2018**
(21) Application number: 10731533.5
(22) Date of filing: 14.07.2010
(51) Int. Cl.: A61J 1/10, A61M 11/02, A61M 5/148

(54) **MEDICAMENT CONTAINER**
ARZNEIMITTELBEHÄLTER
CONTENEUR DE MÉDICAMENTS

(30) Priority: 14.07.2009 EP 09009188
(43) Date of publication of application: 23.05.2012
(73) Proprietor: Sanofi-Aventis Deutschland GmbH, 65929 Frankfurt am Main (DE)
(72) Inventor: NAGEL, Thomas, 01737 Tharandt (DE); RICHTER, René, 01737 Tharandt (DE); WITT, Robert, 01159 Dresden (DE)
(74) Representative: Finger, Catrin
(86) International application number: PCT/EP2010/060125
(87) International publication number: WO 2011/006923

(56) References cited:
- EP-A1- 2 042 207
- US-A- 2 805 662
- US-A- 5 330 431
- US-A- 5 368 199
- US-A1- 2001 016 710
- US-A1- 2005 277 887

## Description

The invention relates to a medicament container, comprising a bag with an outlet, the bag compressible by a compression means.

Many medicaments have to be injected into the body. This applies in particular to medicaments, which are deactivated or have their efficiency remarkably decreased by oral administration, e.g. proteines (such as insulin, growth hormones, interferons), carbohydrates (e.g. heparin), antibodies and the majority of vaccines. Such medicaments are predominantly injected by means of syringes, medicament pens or medicament pumps.

Some medicaments have to be administered by inhaling them from so called inhalers.

WO 2009/069518 A1 discloses an inhaler, wherein the medicament to be inhaled is stored in a bag shaped medicament container.

US 5 330 431 discloses an infusion pump with a medicament container and a compression means, the medicament container comprising a bag with an outlet, the bag being compressible by said compression means. The bag is flexible.

US 2001/016710 discloses a medication delivery device with a medicament container and a compression means, the medicament container comprising a bag with an outlet, the bag being compressible by said compression means. The bag is flexible.

US 2 805 662 discloses a medicament container and a compression means, the medicament container comprising a bag with an outlet, the bag being compressible by said compression means. The bag has an inflexible lower half and a flexible upper half. The flexible upper half is intended to be subjected to the compression means.

EP 2 042 207 discloses a portable drug solution container for throat drug solutions and a compression means, the drug solution container comprising a bag with an outlet, the bag being compressible by said compression means. The bag is flexible.

US 2005/277887 discloses an infusion pump with a medicament container and a compression means, the medicament container comprising a bag with an outlet, the bag being compressible by said compression means. The bag is flexible.

US 5 368 199 discloses a container for holding a hot melt material. The container comprises a susceptor layer, a microwave transparent layer and a heat transmissive and microwace transparent layer. The contained hot melt material is softened by heat generated by microwave radiation.

It is an object of the present invention to provide an improved medicament container.

It is a further object of the present invention to provide an injection arrangement which avoids delayed dripping and improves dose accuracy.

The object is achieved by a medicament container according to claim 1.

Preferred embodiments of the invention are given in the dependent claims.

An injection arrangement with a medicament container according to the invention comprises a bag with an outlet. The bag, preferably having a relatively thin skin, is compressible by a compression means so as to gradually reducing a volume of the bag and consequently squeezing the medicament stored in the bag out of the outlet similar to a tube of tooth paste. Since the outlet usually exhibits some kind of bottleneck, in particular when equipped with valves and/or injection needles, compression of the bag results in an internal pressure inside the bag. The bag is arranged to be flexible or soft in an area respectively subjected to the compression means and arranged to be essentially inflexible in the remaining areas and in the areas that have not yet been subjected to the compression means. This behaviour avoids a temporary expansion of the bag in the areas not subjected to the compression means due to the increased internal pressure so the effort expended in compressing the bag goes virtually entirely in the delivery of the medicament through the outlet rather than in expansion of the bag. The inventive arrangement avoids delayed dripping due to relaxation of the expanded bag when the advancement of the compression means is stopped. Furthermore, dosing may be performed with an improved precision.

The bag may comprise a thermally sensitive skin or skin layer, which is arranged for turning flexible upon subjection to a heated compression means and for being essentially inflexible when not heated or when cooled down after heating. This applies in particular to an outer skin layer which is in direct contact with the compression means rather than with the liquid content.

In another embodiment of the invention the bag comprises a skin or skin layer, that may be locally softened or turned flexible by subjection to at least one of a chemical agent, a radiation, a magnetic field, an electrical field, an electrical current and a mechanical influence. This applies in particular to an outer skin layer which is in direct contact with the compression means rather than with the liquid content.

In yet another embodiment the bag may comprise a skin with at least one flexible layer and a stiff outer skin layer, which is removable when subjected to the compression means.

The skin layer may comprise one of sugar, salt and wax which are essentially inflexible materials.

The skin or skin layer may also comprise silver, e.g. in the shape of silver ions, wherein the skin or skin layer is arranged for being turned flexible or wherein the skin layer is arranged for being removed or dissolved by subjection to radiation with visible light or ultraviolet light. It is known from black and white photographic processing that properties of silver-bearing substances may be altered by subjecting them to radiation. The skin or skin layer may also comprise a UV sensitive polymer, wherein the skin or skin layer is arranged for being turned flexible or wherein the skin layer is arranged for being removed or dissolved by subjection to radiation with ultraviolet light as known from wafer exposure.

In a preferred embodiment of the invention the bag is arranged on an essentially planar, rigid support and a roller is arranged for locally pressing the bag against the support and advancing in a longitudinal direction of the bag. The roller may alternatively be replaced by a shoe or wiper pressed against the bag and advancing without being rotated.

The medicament container may be part of an injection arrangement or an inhaler arrangement for delivering a liquid medicament to a human or an animal.

The injection arrangement may comprise a valve and a hollow needle for piercing a patient's skin, the valve and needle being arranged at the outlet of the medicament container. In case of a jet injector, instead of the needle, a jet nozzle may be arranged.

The medicament container may preferably be used for delivering one of an analgetic, an anticoagulant, insulin, an insulin derivate, heparin, Lovenox, a vaccine, a growth hormone, a peptide hormone, a proteine, and complex carbohydrates.

The skin or outer skin layer may also improve protection of the bag's contents against ambient influences.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

The present invention will become more fully understood from the detailed description given hereinbelow and the accompanying drawings which are given by way of illustration only, and thus, are not limitive of the present invention, and wherein:

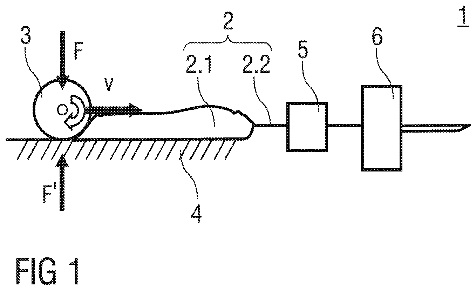

Figure 1 is a schematic view of an injection arrangement comprising a medicament container, a compression means and a support.
Figure 1 shows a schematic view of an injection arrangement 1 comprising a medicament container 2, a compression means 3 and a support 4.

The medicament container 2 comprises a bag 2.1 with an outlet 2.2. The bag 2.1 has a relatively thin skin and is compressible by the compression means 3. Thereby a volume of the bag 2.1 is gradually reduced and consequently a medicament stored in the bag 2.1 squeezed out of the outlet 2.2. The injection arrangement 1 further comprises a valve 5 and a hollow needle 6 arranged at the outlet 2.2. The compression means 3 is in the shape of a roller which may be pressed with a force F against the support 4 which exerts a counteracting force F'. Due to this action the bag 2.1 is locally compressed between the compression means 3 and the support. The compression means may then be advanced with a speed v in the direction indicated by the arrow assigned to v, thereby gradually reducing the bag's 2.1 volume.

When the bag 2.1 is compressed an internal pressure inside the bag 2.1 increases. The bag 2.1 is arranged to be flexible or soft in an area respectively subjected to the compression means 3 and arranged to be essentially inflexible in the remaining areas or in the areas that have not yet been subjected to the compression means 3.

The bag 2.1 may comprise a thermally sensitive skin or skin layer, which is arranged for turning flexible upon subjection to a heated compression means 3 and for being essentially inflexible when not heated or cooled down after heating. This applies in particular to on outer skin layer which is in direct contact with the compression means 3 rather than with the liquid content.

In another embodiment of the invention the bag 2.1 comprises a skin or skin layer, that may be locally softened or turned flexible by subjection to at least one of a chemical agent, a radiation, a magnetic field, an electrical field, an electrical current and a mechanical influence. The subjection to the respective medium may be exerted by the compression means 3 or by a separate arrangement advancing with the compression means 3.

In yet another embodiment the bag 2.1 may comprise a skin with at least one flexible layer and a stiff outer skin layer, which is removable when or before subjected to the compression means 3. E.g. the skin layer may be peeled of or unwound from the skin by means of the compression means 3 or another arrangement travelling ahead of the compression means 3.

The skin layer may comprise one of sugar, salt and wax.

The skin or skin layer may also comprise silver, e.g. in the shape of silver ions, wherein the skin or skin layer is arranged for being turned flexible or wherein the skin layer is arranged for being removed or dissolved by subjection to radiation with visible light or ultraviolet light.

The compression means 3 in the shape of the roller may alternatively be replaced by a shoe or wiper pressed against the bag 2.1 and advancing without being rotated.

The medicament container may be part of any kind of an injection arrangement or an inhaler arrangement for delivering a liquid medicament to a human or an animal.

Instead of the hollow needle 6 shown in the figure a jet nozzle may be provided.

The medicament container 2 may preferably be used for delivering one of an analgetic, an anticoagulant, insulin, an insulin derivate, heparin, Lovenox, a vaccine, a growth hormone, a peptide hormone, a proteine, and complex carbohydrates.

The term "medicament", as used herein, means a pharmaceutical formulation containing at least one pharmaceutically active compound,

wherein in one embodiment the pharmaceutically active compound has a molecular weight up to 1500 Da and/or is a peptide, a proteine, a polysaccharide, a vaccine, a DNA, a RNA, a antibody, an enzyme, an antibody, a hormone or an oligonucleotide, or a mixture of the above-mentioned pharmaceutically active compound,

wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,

wherein in a further embodiment the pharmaceutically active compound comprises at least one peptide for the treatment and/or prophylaxis of diabetes mellitus or complications associated with diabetes mellitus such as diabetic retinopathy,

wherein in a further embodiment the pharmaceutically active compound comprises at least one human insulin or a human insulin analogue or derivative, glucagon-like peptide (GLP-1) or an analogue or derivative thereof, or exedin-3 or exedin-4 or an analogue or derivative of exedin-3 or exedin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
   H-(Lys)5-des Pro36, des Pro37 Exendin-4(1-39)-NH2,
   des Pro36 [Asp28] Exendin-4(1-39),
   des Pro36 [IsoAsp28] Exendin-4(1-39),
   des Pro36 [Met(O)14, Asp28] Exendin-4(1-39),
   des Pro36 [Met(O)14, IsoAsp28] Exendin-4(1-39),
   des Pro36 [Trp(O2)25, Asp28] Exendin-4(1-39),
   des Pro36 [Trp(O2)25, IsoAsp28] Exendin-4(1-39),
   des Pro36 [Met(O)14 Trp(O2)25, Asp28] Exendin-4(1-39),
   des Pro36 [Met(O)14 Trp(O2)25, IsoAsp28] Exendin-4(1-39); or
des Pro36 [Asp28] Exendin-4(1-39),
   des Pro36 [IsoAsp28] Exendin-4(1-39),
   des Pro36 [Met(O)14, Asp28] Exendin-4(1-39),
   des Pro36 [Met(O)14, IsoAsp28] Exendin-4(1-39),
   des Pro36 [Trp(O2)25, Asp28] Exendin-4(1-39),
   des Pro36 [Trp(O2)25, IsoAsp28] Exendin-4(1-39),
   des Pro36 [Met(O)14 Trp(O2)25, Asp28] Exendin-4(1-39),
   des Pro36 [Met(O)14 Trp(O2)25, IsoAsp28] Exendin-4(1-39),
   wherein the group -Lys6-NH2 may be bound to the C-terminus of the Exendin-4 derivative;
or an Exendin-4 derivative of the sequence
H-(Lys)6-des Pro36 [Asp28] Exendin-4(1-39)-Lys6-NH2,
   des Asp28 Pro36, Pro37, Pro38Exendin-4(1-39)-NH2,
   H-(Lys)6-des Pro36, Pro38 [Asp28] Exendin-4(1-39)-NH2,
   H-Asn-(Glu)5des Pro36, Pro37, Pro38 [Asp28] Exendin-4(1-39)-NH2,
   des Pro36, Pro37, Pro38 [Asp28] Exendin-4(1-39)-(Lys)6-NH2,
   H-(Lys)6-des Pro36, Pro37, Pro38 [Asp28] Exendin-4(1-39)-(Lys)6-NH2,
   H-Asn-(Glu)5-des Pro36, Pro37, Pro38 [Asp28] Exendin-4(1-39)-(Lys)6-NH2,
   H-(Lys)6-des Pro36 [Trp(O2)25, Asp28] Exendin-4(1-39)-Lys6-NH2,
   H-des Asp28 Pro36, Pro37, Pro38 [Trp(O2)25] Exendin-4(1-39)-NH2,
   H-(Lys)6-des Pro36, Pro37, Pro38 [Trp(O2)25, Asp28] Exendin-4(1-39)-NH2,
   H-Asn-(Glu)5-des Pro36, Pro37, Pro38 [Trp(O2)25, Asp28] Exendin-4(1-39)-NH2,
   des Pro36, Pro37, Pro38 [Trp(O2)25, Asp28] Exendin-4(1-39)-(Lys)6-NH2,
   H-(Lys)6-des Pro36, Pro37, Pro38 [Trp(O2)25, Asp28] Exendin-4(1-39)-(Lys)6-NH2,
   H-Asn-(Glu)5-des Pro36, Pro37, Pro38 [Trp(O2)25, Asp28] Exendin-4(1-39)-(Lys)6-NH2,
   H-(Lys)6-des Pro36 [Met(O)14, Asp28] Exendin-4(1-39)-Lys6-NH2,
   des Met(O)14 Asp28 Pro36, Pro37, Pro38 Exendin-4(1-39)-NH2,
   H-(Lys)6-desPro36, Pro37, Pro38 [Met(O)14, Asp28] Exendin-4(1-39)-NH2,
   H-Asn-(Glu)5-des Pro36, Pro37, Pro38 [Met(O)14, Asp28] Exendin-4(1-39)-NH2,
   des Pro36, Pro37, Pro38 [Met(O)14, Asp28] Exendin-4(1-39)-(Lys)6-NH2,
   H-(Lys)6-des Pro36, Pro37, Pro38 [Met(O)14, Asp28] Exendin-4(1-39)-(Lys)6-NH2,
   H-Asn-(Glu)5 des Pro36, Pro37, Pro38 [Met(O)14, Asp28] Exendin-4(1-39)-(Lys)6-NH2,
   H-Lys6-des Pro36 [Met(O)14, Trp(O2)25, Asp28] Exendin-4(1-39)-Lys6-NH2,
   H-des Asp28 Pro36, Pro37, Pro38 [Met(O)14, Trp(O2)25] Exendin-4(1-39)-NH2,
   H-(Lys)6-des Pro36, Pro37, Pro38 [Met(O)14, Asp28] Exendin-4(1-39)-NH2,
   H-Asn-(Glu)5-des Pro36, Pro37, Pro38 [Met(O)14, Trp(O2)25, Asp28] Exendin-4(1-39)-NH2,
   des Pro36, Pro37, Pro38 [Met(O)14, Trp(O2)25, Asp28] Exendin-4(1-39)-(Lys)6-NH2,
   H-(Lys)6-des Pro36, Pro37, Pro38 [Met(O)14, Trp(O2)25, Asp28] Exendin-4(S1-39)-(Lys)6-NH2,
   H-Asn-(Glu)5-des Pro36, Pro37, Pro38 [Met(O)14, Trp(O2)25, Asp28] Exendin-4(1-39)-(Lys)6-NH2;
or a pharmaceutically acceptable salt or solvate of any one of the afore-mentioned Exedin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Pharmaceutically acceptable salts are for example acid addition salts and basic salts. Acid addition salts are e.g. HCI or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

### List of References

- 1: injection arrangement
- 2: medicament container
- 2.1: bag
- 2.2: outlet
- 3: compression means
- 4: support
- 5: valve
- 6: hollow needle
- F: force
- F': counteracting force
- v: speed

## Claims

1. Medicament container (2) in combination with a compression means (3), the medicament container (2) comprising a bag (2.1) with an outlet (2.2), the bag (2.1) being compressible by said compression means (3), **characterized in that** the bag (2.1) skin is made of a material which is essentially inflexible and is arranged for turning flexible in an area subjected to said compression means (3) in order to avoid temporary expansion of the bag (2.1) in the areas not subjected to the compression means (3) due to the increased internal pressure during bag (2.1) compression.

2. Medicament container (2) according to claim 1, **characterized in that** the bag (2.1) is arranged to return inflexible in areas that have already been subjected to the compression means (3).

3. Medicament container (2) according to claim 2, **characterized in that** the bag (2.1) comprises a thermally sensitive skin or skin layer, which is arranged for turning flexible upon subjection to a heated compression means (3) and for being essentially inflexible when not heated or cooled down after heating.

4. Medicament container (2) according to one of the claims 1 or 2, **characterized in that** the bag (2.1) comprises a skin or skin layer, that may be locally softened or turned flexible by subjection to at least one of a chemical agent, a radiation, a magnetic field, an electrical field, an electrical current, a heated compression means and a mechanical influence.

5. Medicament container (2) according to one of the claims 3 or 4, **characterized in that** the skin layer is an outer layer.

6. Medicament container (2) according to claim 1, **characterized in that** the bag (2.1) comprises a skin with at least one flexible layer and a stiff outer skin layer, which is removable when subjected to the compression means (3).

7. Medicament container (2) according to one of the claims 4 to 6, **characterized in that** the skin layer comprises one of sugar, salt and wax.

8. Medicament container (2) according to one of the claims 4 to 7, **characterized in that** the skin or skin layer comprises silver or a UV sensitive polymer, wherein the skin or skin layer is arranged for being turned flexible or wherein the skin layer is arranged for being removed or dissolved by subjection to radiation with visible light or ultraviolet light.

9. Medicament container (2) according to one of the claims 1 to 8, **characterized in that** the bag (2.1) is arranged on an essentially planar, rigid support (4) and a roller serving as the compression means (3) is arranged for locally pressing the bag (2.1) against the support (4) and advancing in a longitudinal direction of the bag (2.1).

10. Injection arrangement (1) for delivering a liquid medicament comprising a medicament container (2) according to one of the claims 1 to 9.

11. Injection arrangement (1) according to claim 10, **characterized in that** a valve (5) and a hollow needle (6) for piercing a patient's skin are arranged at the outlet (2.2).

12. Inhaler arrangement for delivering a liquid medicament comprising a medicament container (2) according to one of the claims 1 to 9.

## Patentansprüche

1. Arzneimittelbehälter (2) in Kombination mit einem Kompressionsmittel (3), wobei der Arzneimittelbehälter (2) einen Beutel (2.1) mit einem Auslass (2.2) umfasst, wobei der Beutel (2.1) vom Kompressionsmittel (3) zusammengedrückt werden kann, **dadurch gekennzeichnet, dass** die Außenhaut des Beutels (2.1) aus einem Material besteht, das im Wesentlichen unflexibel ist und das ausgebildet ist, in einem Bereich, der dem Kompressionsmittel (3) ausgesetzt ist, flexibel zu werden, um eine temporäre Ausdehnung des Beutels (2.1) in den Bereichen, die dem Kompressionsmittel (3) nicht ausgesetzt sind, aufgrund von erhöhtem Innendruck während der Kompression des Beutels (2.1) zu vermeiden.

2. Arzneimittelbehälter (2) nach Anspruch 1, **dadurch gekennzeichnet, dass** der Beutel (2.1) ausgebildet ist, in Bereichen, die bereits dem Kompressionsmittel (3) ausgesetzt wurden, wieder unflexibel zu werden.

3. Arzneimittelbehälter (2) nach Anspruch 2, **dadurch gekennzeichnet, dass** der Beutel (2.1) eine wärmeempfindliche Außenhaut oder Hautschicht umfasst, die angeordnet ist, flexibel zu werden, wenn sie einem erwärmten Kompressionsmittel (3) ausgesetzt wird, und im Wesentlichen unflexibel zu sein, wenn sie nicht erwärmt wird oder nach dem Erwärmen abgekühlt wird.

4. Arzneimittelbehälter (2) nach einem der Ansprüche 1 oder 2, **dadurch gekennzeichnet, dass** der Beutel (2.1) eine Außenhaut oder Hautschicht umfasst, die örtlich erweicht oder flexibel gemacht werden kann, indem sie mindestens einem von einem chemischen Mittel, einer Strahlung, einem Magnetfeld, einem elektrischen Feld, einem elektrischen Strom, einem erwärmten Kompressionsmittel und einem mechanischem Einfluss ausgesetzt wird.

5. Arzneimittelbehälter (2) nach einem der Ansprüche 3 oder 4, **dadurch gekennzeichnet, dass** die Hautschicht eine Außenschicht ist.

6. Arzneimittelbehälter (2) nach Anspruch 1, **dadurch gekennzeichnet, dass** der Beutel (2.1) eine Außenhaut mit mindestens einer flexiblen Schicht und einer steifen äußeren Hautschicht umfasst, die entfernbar ist, wenn sie dem Kompressionsmittel (3) unterworfen wird.

7. Arzneimittelbehälter (2) nach einem der Ansprüche 4 bis 6, **dadurch gekennzeichnet, dass** die Hautschicht eines von Zucker, Salz und Wachs umfasst.

8. Arzneimittelbehälter (2) nach einem der Ansprüche 4 bis 7, **dadurch gekennzeichnet, dass** die Außenhaut oder Hautschicht Silber oder ein UV-empfindliches Polymer umfasst, wobei die Außenhaut oder Hautschicht angeordnet ist, flexibel gemacht zu werden, oder wobei die Hautschicht angeordnet ist, entfernt oder aufgelöst zu werden, indem sie Strahlung mit sichtbarem Licht oder Ultraviolettlicht ausgesetzt wird.

9. Arzneimittelbehälter (2) nach einem der Ansprüche 1 bis 8, **dadurch gekennzeichnet, dass** der Beutel (2.1) auf einer im Wesentlichen ebenflächigen, starren Stütze (4) angeordnet ist und eine als Kompressionsmittel (3) dienende Rolle dazu angeordnet ist, den Beutel (2.1) örtlich gegen die Stütze (4) zu drücken, und in einer Längsrichtung des Beutels (2.1) vorgeschoben zu werden.

10. Injektionsanordnung (1) zur Verabreichung eines flüssigen Medikaments, umfassend einen Arzneimittelbehälter (2) nach einem der Ansprüche 1 bis 9.

11. Injektionsanordnung (1) nach Anspruch 10, **dadurch gekennzeichnet, dass** ein Ventil (5) und eine Hohlnadel (6) zum Einstechen in die Haut eines Patienten am Auslass (2.2) angeordnet sind.

12. Inhalatoranordnung zur Verabreichung eines flüssigen Medikaments, umfassend einen Arzneimittelbehälter (2) nach einem der Ansprüche 1 bis 9.

## Revendications

1. Récipient (2) pour médicament en combinaison avec un moyen de compression (3), le récipient pour médicament (2) comprenant une poche (2.1) avec une sortie (2.2), la poche (2.1) pouvant être comprimée par le moyen de compression (3),
**caractérisé en ce que** la peau de la poche (2.1) est faite d'une matière pour l'essentiel rigide et est agencée pour devenir souple dans la zone soumise audit moyen de compression (3) afin d'éviter une expansion temporaire de la poche (2.1) dans les zones non soumises au moyen de compression (3) en raison de l'augmentation de la pression interne pendant la compression de la poche (2.1).

2. Récipient (2) pour médicament selon la revendication 1, **caractérisé en ce que** la poche (2.1) est agencée pour redevenir rigide dans les zones qui ont déjà été soumises au moyen de compression (3).

3. Récipient (2) pour médicament selon la revendication 2, **caractérisé en ce que** la poche (2.1) comprend une peau ou une couche de peau thermosensible, qui est agencée pour devenir souple lorsqu'on la soumet à un moyen de compression (3) chauffé et pour être essentiellement rigide quand elle n'est pas chauffée ou refroidie après chauffage.

4. Récipient (2) pour médicament selon la revendication 1 ou 2, **caractérisé en ce que** la poche (2.1) comprend une peau ou une couche de peau qu'on peut localement ramollir ou rendre souple en la soumettant à au moins un facteur parmi agent chimique, rayonnement, champ magnétique, champ électrique, courant électrique, moyen de compression chauffé et influence mécanique.

5. Récipient (2) pour médicament selon la revendication 3 ou 4, **caractérisé en ce que** la couche de peau est une couche extérieure.

6. Récipient (2) pour médicament selon la revendication 1, **caractérisé en ce que** la poche (2.1) comprend une peau avec au moins une couche souple et une couche de peau extérieure raide, qui peut être retirée quand on la soumet au moyen de compression (3).

7. Récipient (2) pour médicament selon l'une quelconque des revendications 4 à 6, **caractérisé en ce que** la couche de peau est constituée de sucre, de sel ou de cire.

8. Récipient (2) pour médicament selon l'une quelconque des revendications 4 à 7, **caractérisé en ce que** la peau ou couche de peau comprend de l'argent ou un polymère sensible aux UV, dans lequel la peau ou couche de peau est agencée pour être assouplie ou dans lequel la couche de peau est agencée pour être retirée ou dissoute par soumission à un rayonnement de lumière visible ou de lumière ultraviolette.

9. Récipient (2) pour médicament selon l'une quelconque des revendications 1 à 8, **caractérisé en ce que** la poche (2.1) est disposée sur un support rigide (4) sensiblement plan et un rouleau servant de moyen de compression (3) est agencé pour presser localement la poche (2.1) contre le support (4) et avancer dans le sens longitudinal de la poche (2.1).

10. Agencement d'injection (1) pour administrer un médicament liquide, comprenant un récipient (2) pour médicament selon l'une quelconque des revendications 1 à 9.

11. Agencement d'injection (1) selon la revendication 10, **caractérisé en ce qu'**un clapet (5) et une aiguille creuse (6) pour percer la peau d'un patient sont disposés à la sortie (2.2).

12. Agencement d'inhalateur pour administrer un médicament liquide, comprenant un récipient (2) pour médicament selon l'une quelconque des revendications 1 à 9.
